# FASCICULE DE BREVET EUROPEEN

(11) **EP 1 740 536 B1**
(45) Date de publication et mention de la délivrance du brevet: **30.11.2016**
(21) Numéro de dépôt: 05717538.2
(22) Date de dépôt: 03.02.2005
(51) Int. Cl.: C07C 323/60, C07C 233/13, C07C 233/36, A62D 1/02

(54) **NOUVEAUX AGENTS TENSIOACTIFS PERFLUOROALKYLES , HYDROPHOBES ET OLEOPHOBES DERIVES D"AMIDES SUBSTITUEES, LEUR PREPARATION ET LEUR UTILISATION**
NEUE HYDROPHOBE UND ÖLABWEISENDE, AUS SUBSTITUIERTEN AMIDEN GEWONNENE PERFLUORALKYLTENSIDE UND DEREN HERSTELLUNG UND VERWENDUNG
NOVEL PERFLUOROALKYL HYDROPHOBIC AND OIL-REPELLENT SURFACTANTS DERIVED FROM SUBSTITUTED AMIDES, PREPARATION AND USE THEREOF

(30) Priorité: 27.02.2004 FR 0401979
(43) Date de publication de la demande: 10.01.2007
(73) Titulaire: SZÖNYI, Stéphane, 06000 Nice (FR); SZÖNYI, Istvan, 98000 Monaco (MC); SZÖNYI, François, 98000 Monaco (MC)
(72) Inventeur: SZÖNYI, Stéphane, 06000 Nice (FR); SZÖNYI, Istvan, 98000 Monaco (MC); SZÖNYI, François, 98000 Monaco (MC)
(86) Numéro de dépôt international: PCT/FR2005/000226
(87) Numéro de publication internationale: WO 2005/092848

(56) Documents cités:
- EP-A- 0 017 568
- DE-A- 2 749 331
- FR-A- 2 103 669
- FR-A- 2 185 668
- FR-A- 2 207 187
- FR-A- 2 575 165
- FR-A- 2 839 071
- US-A- 4 090 967

## Description

La présente invention concerne de nouveaux tensioactifs perfluoroalkylés de type cationique (oxyde d'amine), anionique (sels de carboxylate), amphotère (carboxybétaïne et sulfobétaïne) dont le groupement perfluoré est issu de la télomérisation du tétrafluoroéthylène (TFE) et dont la structure moléculaire correspond à la formule générale suivante :

**R_{F}-A-B-D**

Ces composés ont la particularité de comporter, dans la partie **D,** un groupement amide substitué

**-(CH₂)ₚ-NH-CO-** ou **-(CH₂)_{q}-CO-N(R₃)-**

directement lié ou séparé de la chaîne polyfluoroalkylée par un groupement **-S-(CH₂)_{q}-,**

**-NH(CH₂)_{q}-, -⁺N(R₁)(R₂)-(CH₂)ₚ-** (partie **B).**

Dans cette formule générale :
**R_{F}** est une chaîne perfluoroalkyle linéaire ou ramifiée **CₙF₂ₙ₊₁** où n = 4 à 20, ou un mélange de groupements perfluoroalkyle linéaires ou ramifiés ;
A est un groupement **-CH₂CH(I)CH₂-, -CH=CHCH₂-, -CH₂CH(I)CH₂OCH₂CH(OH) CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-** ou **-C₂H₄S-C₃H₆OCH₂CH(OH)CH₂- ;**
**B** est un groupement **-S-(CH₂)_{q}-, -NH(CH₂)_{q} -** ou **-N**⁺**R₁R₂-(CH₂)ₚ-** lorsque **A** est un groupement **-CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH) CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-,** ou néant lorsque A = **-CH₂CHICH₂-, -CH=CHCH₂- ;**
**D** est un groupement - **CONH-(CH₂)ᵣN⁺R₁R₂-E, -CONR₃(CH₂)ₛCOOM** lorsque **B** est un groupement **-S-(CH₂)_{q}-, -NH(CH₂)_{q}-,**
un groupement **-NHCO-(CH₂)ᵣCOO⁻, -NHCO-CH(SO₃M)CH₂COO,**
lorsque **B = -N⁺R₁R₂-(CH₂)ₚ-,**
un groupement **-NHCO-(CH₂)ᵣCOOM, NHCO-CH(SO₃M)CH₂COOM,**
lorsque **B** est **néant ;**
**E** est un groupement **-(CH₂)ₛCOO⁻, -O⁻, -CH₂CHOHCH₂SO₃ ;**
**M** est un ion alcalin ;
**p** et **r** sont des nombres entiers valant 2 ou 3 ;
**q** et **s** sont des nombres entiers valant 1 ou 2 ;
**R₁** et **R₂** sont des groupements alkyle mineur identiques;
**R₃** est égal à **-H** ou **-CH₃** ;

De nombreux agents tensioactifs perfluoroalkylés de l'art antérieur ont été décrits dans les brevets FR 2.035.589, FR 2.103.669, FR 2.477.144, EP 149.347 (oxydes d'amines), FR 2.088.941, FR 2.185.668, DE 2.559.189, FR 2.390.426, US 4.069.244, US 4.126.633, US 4.283.533 , US 4.795.590 (carboxybétaïnes) ; FR 2.207.187, EP 17.568 (sulfobétaïnes).

Les carboxybétaïnes, sulfobétaïnes, oxydes d'amine et carboxylates perfluoroalkylés de cette invention sont caractérisés par leur grand pouvoir de réduction de la tension superficielle de l'eau et des solutions d'électrolytes inorganiques. En raison de l'abaissement important de la tension superficielle que les composés de l'invention produisent dans l'eau, ils peuvent être utilisés comme agents mouillants, moussants, émulsifiants et dispersants.

Les composés de type carboxybétaïne ou sulfobétaïne sont particulièrement intéressants dans la mesure où ils peuvent être utilisés aussi bien dans les milieux fortement acides que basiques. Les agents tensioactifs perfluoroalkylés de l'invention sont particulièrement efficaces lorsqu'ils sont utilisés comme additifs pour les mousses extinctrices synthétiques (AFFF) et protéiniques (FFFP) filmogènes destinées à la lutte contre les incendies d'hydrocarbures ou de solvants. Les mousses extinctrices ont été décrites dans de nombreux brevets: US 4.042.522, US 4.090.967, DE 2.325.855, DE 2.732.555, DE 2.749.331, DE 2.829.594, EP 311.570, US 5.085.786, EP 780.141 (AFFF); US 3.475.333, DE 1.928.556, DE 2.240.263, FR 2.575.165 (FFFP).

L'originalité de cette invention réside dans le fait que
- les réactions permettant d'obtenir les tensioactifs perfluoroalkylés de l'invention sont faciles et rapides à mettre en oeuvre,
- les composés de l'invention sont aisément synthétisés à partir de produits disponibles dans le commerce par des procédés économiquement valables,
- les composés de l'invention sont préparés sans avoir à séparer et purifier chaque intermédiaire,
- les composés de l'invention ne comportent aucun groupement perfluorooctane sulfonyle ou perfluorooctane carboxyle susceptible de se dégrader respectivement en perfluorooctane sulfonate (PFOS) ou perfluorooctane acide carboxylique (PFOA). Il faut rappeler que les tensioactifs fluorés possédant ce type de groupement dans leur structure moléculaire font actuellement l'objet d'études sur leur éventuelle Persistance, Bioaccumulation et Toxicité (PBT).

Dans le brevet FR 2.839.071, les mêmes auteurs ont décrit auparavant de nouveaux agents tensioactifs perfluoroakylés alcooléophobes dont la structure moléculaire ne contenait déjà aucun groupement **SO₂** lié directement à la chaîne perfluorée.

Les tensioactifs perfluoroalkylés de la présente invention sont préparés
- soit à partir d'époxydes perfluoroalkylés tels que : 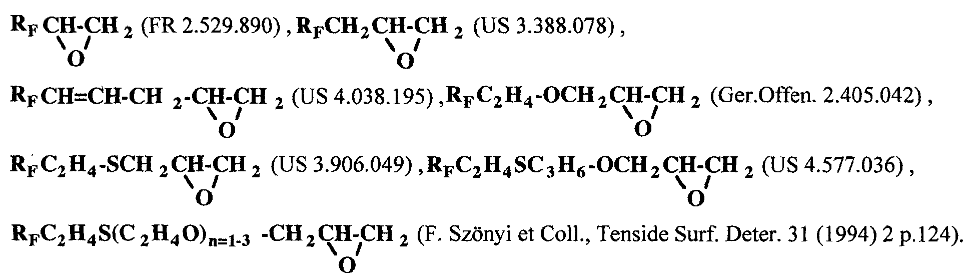
- soit à partir d'époxydes possédant une double liaison terminale comme le groupement allyle du 1-allyloxy-2,3-époxypropane soit encore à partir de l'allylamine . Dans le cas des époxydes, le cycle est ouvert :
- soit par une amine tertiaire qui est obtenue par réaction d'une N,N-dialkylènediamine, qui peut être la N,N-diméthyléthylènediamine, la N,N-diéthyléthylènediamine, et de préférence la N,N-diméthylpropylènediamine ou la N,N-diéthylpropylènediamine avec un anhydride d'acide tel que l'acide succinique, glutarique, maléique. Si l'anhydride d'acide est l'anhydride maléique, sa double liaison peut-être préalablement soumise à une réaction de sulfonation au moyen de bisulfite de potassium ;
- soit par un α-thioester tel que le glycolate de méthyle ou d'éthyle ou par un β-thioester tel que le 3-mercaptopropionate de méthyle ou d'éthyle;
- soit par un α-aminoester tel que le glycinate de méthyle ou d'éthyle ou par un β-aminoester tel que le 3-aminopropionate de méthyle ou d'éthyle. Les intermédiaires issus de la condensation des thioesters et des aminoesters sont amidés soit par un par un α-aminoacide tel que la glycine ou la sarcosine, soit par un β-aminoacide tel que la β-alanine ou par une diamine [par exemple: **H₂N(CH₂)ₚN(CH₃)₂].**

Dans le cas de l'allylamine, celle-ci est mise à réagir avec un anhydride d'acide tel que l'acide succinique, glutarique, maléique. Les doubles liaisons de type allylique, lorsqu'elles sont contenues dans les molécules obtenues, sont alors perfluoroalkylées selon les règles de l'art. On utilise comme agent perfluoroalkylant les iodures de perfluoroalcane **CₙF₂ₙ₊₁I** qui sont des matières premières obtenues par le procédé de télomérisation du tétrafluoroéthylène illustré dans "Fluorocarbons and their Derivatives" par R.E.Banks, London, 1964, p.56-61. Les iodures de perfluoroalcane **CₙF₂ₙ₊₁I** dans lesquels **CₙF₂ₙ₊₁** est un groupement perfluoroalkyle linéaire ou ramifié où n est un nombre entier valant 4 à 20, ou un mélange de groupements perfluoroalkyles linéaires ou ramifiés, réagissent avec les doubles liaisons terminales soit en présence de radicaux libres de type azoïque ou peroxyde, selon des procédés bien connus (brevet US 3.145.222), soit par action simultanée de ces initiateurs de radicaux libres et de sel de dithionite (W.Y.Hung, J.Fluorine chem. 58 (1992) 1-8), de bisulfite (W.Y.Hung, J.Zhuang, Chin.J.Chem. 9(1991) 373), ou de dioxyde de thiourée (W.Y.Hung, J.Zhuang, Chin.J.Chem., 9(1991) 270).

Les tensioactifs amphotères de type carboxybétaïne correspondant à :
**A = CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂- B = -⁺N(R₁)(R₂)-(CH₂)ₚ-**
avec R₁ et R₂ = Me ou Et.

**D = -NHCO-(CH₂)ᵣCOO⁻, -NHCO-CH(SO₃M)CH₂COO,**

avec r = 2 ou 3
sont préparés en trois étapes :
On fait d'abord réagir dans la N,N-diméthylformamide une diamine de formule **H₂N-(CH₂)ₚ-N(R₁)(R₂)** où p = 2 ou 3, R₁ et R₂ = groupements alkyle mineur, avec un anhydride d'acide qui peut être l'anhydride succinique, glutarique, maléique.

Le composé qui précipite est le N,N-dialkylaminoamidoacide de formule :
**(R₁)(R₂)N-(CH₂)ₚD-H *(I)*** où **D** est défini ci-dessus (dans le cas où **D = -NH-COCH=CH-COO-,** avant de procéder à la quaternisation du groupement amine tertiaire **(R₁)(R₂)N-** du composé **(*I*),** la double liaison est soumise à une réaction de sulfonation).

Les composés **(*I*)** sont dissous dans le 2-propanol , puis, afin de quaterniser le groupement amine tertiaire **(R₁)(R₂)N-** on ajoute lentement , soit le 1-allyloxy-2,3-époxypropane pour obtenir les composés de formule suivante :

**CH₂=CH-CH₂OCH₂CH(OH)CH₂-⁺N(R₁)(R₂)-(CH₂)ₚ-D** **(*II*),**

soit un époxyde perfluoroalkylé de formule suivante :

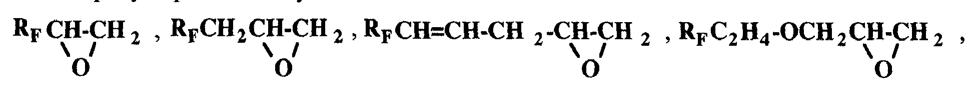

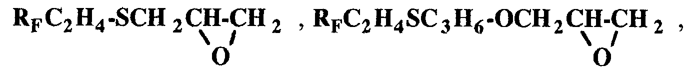

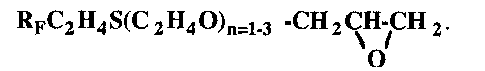

On obtient alors les tensioactifs perfluoroalkylés de type carboxybétaïne selon l'invention, avec la formule suivante :

**R_{F}** - **A-⁺N(R₁)(R₂)-(CH₂)ₚ-D** (III)

avec A = - **CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-** et D tel que défini précédemment,
par perfluoroalkylation de la double liaison allylique du composé ***(II)*** par les iodures de perfluoroalkyle en présence de 2,2'-azobis(isobutyronitrile) et d'hydrosulfite de sodium, ou directement dans le cas de l'utilisation d'époxydes perfluoroalkylés.

Le mélange obtenu est dilué avec une petite quantité de monobutyléther du diéthylène glycol et complété avec de l'eau de telle sorte que la matière active des tensioactifs perfluoroalkylés de type carboxybétaïne de l'invention soit d'environ 30% avec une teneur en fluor d'environ 9%. Le pH du mélange est réglé vers 7 ± 0,5.

Les tensioactifs anioniques de type carboxylate correspondant à :
**A = -CH₂CH(I)CH₂-** ou **CH = CH-CH₂-**
**B =** néant
**D = -NHCO- (CH₂)ᵣCOOM, -NHCO-CH(SO₃M)CH₂COOM,**
avec r = 2 ou 3 sont obtenus en trois étapes :

On fait réagir l'allylamine dans la N,N-diméthylformamide avec un anhydride d'acide qui peut être l'anhydride succinique, glutarique, maléique.

Le composé amidoacide obtenu a pour formule **CH₂=CH-CH₂-D (*IV*)**
où **D** est défini ci-dessus avec **M = H.**

La double liaison du groupement allylique du composé **(*IV*)** est ensuite perfluoroalkylée à l'aide des iodures de perfluoroalcane en présence de 2,2'azobis(isobutyronitrile) et de l'hydrosulfite de sodium. Enfin, en traitant le mélange à l'aide de l'hydroxyde de sodium ou de potassium, on obtient les tensioactifs perfluoroalkylés de type carboxylate de sodium ou de potassium selon l'invention. Le mélange est dilué avec une petite quantité de monobutyléther du diéthylène glycol et complété avec de l'eau de telle sorte que la matière première active des tensioactifs perfluoroalkylés anioniques de type carboxylate de sodium ou de potassium soit d'environ 25% avec une teneur en fluor d'environ 8,7%. Le pH du mélange est réglé vers 7 ± 0,5.

D'autres tensioactifs anioniques de type carboxylate correspondant à
**A = CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-**
**B = -S(CH₂)_{q}-, -NH(CH₂)_{q}-**
**D = -CO-NR₃(CH₂)ₛCOO⁻Na⁺** ou **-CO-NR₃(CH₂)ₛCOO⁻K⁺**
avec R₃ = H ou Me
sont également obtenus en deux ou trois étapes : on fait réagir le 1-allyloxy-2,3 époxypropane ou les époxydes de formules suivantes : 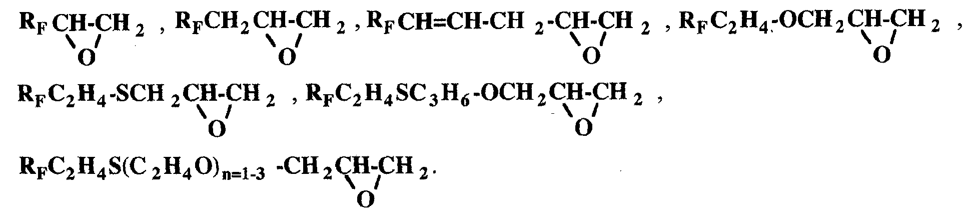 avec le thioglycolate de méthyle ou d'éthyle, le glycinate de méthyle ou d'éthyle, le 3-mercaptopropionate de méthyle ou d'éthyle ou le 3-aminopropionate de méthyle ou d'éthyle.

Les composés amino et thioesters obtenus ont pour formule

**CH₂=CH-CH₂-O-CH₂-CH(OH)CH₂-S(CH₂)_{q}COOR**₅ **(*V*)**

ou

**CH₂=CH-CH₂-O-CH₂-CH(OH)CH₂-NH(CH₂)_{q}COOR₅** ***(VI)***

**R_{F}-A-S(CH₂)_{q}COOR₅** ***(VII)***

ou

**R_{F}-A-NH(CH₂)_{q}COOR₅** **(*VIII*)**

Avec **A = - CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-**
avec q = 1 ou 2 et R₅ = Me ou Et
Par exemple, la fonction ester du composé **(*V*)** est soumise à une réaction de transamidation que l'on effectue avec la glycine, le glycinate de méthyle ou d'éthyle, 3-aminopropionate de méthyle ou d'éthyle ou la sarcosine pour obtenir les composés de formule :

**CH₂=CH-CH₂OCH₂CH(OH)CH₂S(CH₂)_{q}CON(R₃)(CH₂)ₛCOOR₆** (*IX*)

où q = 1 ou 2, s = 1 ou 2, R₆ = H, Me ou Et.

La double liaison du groupement allylique du composé ***(IX)*** est ensuite perfluoroalkylée à l'aide des iodures de perfluoroalcane en présence de 2,2' azobis (isobutyronitrile) et d'hydrosulfite de sodium. Enfin on obtient les tensioactifs perfluoroalkylés anioniques de type carboxylate de sodium ou de potassium de l'invention en traitant le mélange avec de l'hydroxyde de sodium ou de potassium. Le mélange est dilué avec une petite quantité de monobutyl éther de diéthylène glycol et complété avec de l'eau de telle sorte que la matière active des tensioactifs perfluoroalkylés anioniques de type carboxylate de sodium ou de potassium soit d'environ 30% avec une teneur en fluor d'environ 9%.

Le pH du mélange est de 10 ± 0,5

Les tensioactifs perfluoroalkylés de type cationique et amphotère correspondant à :
**A** = **CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-**
**B = -NH-(CH₂)_{q}-**
**D = -CO-NH-(CH₂)ᵣ-⁺N(R₁)(R₂)-O⁻, -(CH₂)ᵣ-⁺N(R₁)(R₂)-(CH₂)ₛCOO⁻, -(CH₂)-⁺N(R₁)(R₂)CH₂CH(OH)CH₂SO₃⁻,**
avec q = 1 ou 2, r = 2 ou 3, s = 1 ou 2 et R₁, R₂ = Me ou Et:
On fait réagir le 1-allyloxy-2,3 époxypropane ou les époxydes de formules suivantes : 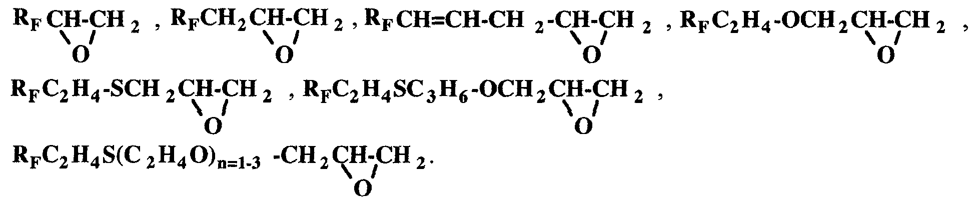 avec le glycinate de méthyle ou d'éthyle ou le 3-aminopropionate de méthyle ou d'éthyle pour obtenir le composé de formule

   **CH₂=CH-CH₂-O-CH₂-CH(OH)CH₂NH(CH₂)_{q}COOR₅** **(*VI*)**

   ou

   **R_{F}-A-NH(CH₂)_{q}COOR₅** ***(VIII)***

   avec
   **A** = **-CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-** et R₅ = Me ou Et, q = 1 ou 2.

La fonction ester des composés **(*VI*)** ou **(*VIII*)** est soumise à une transamidation qui est effectuée avec une diamine comme **H₂N(CH₂)ₚN(alkyle mineur)₂;** à titre d'exemple on peut utiliser la N,N-diméthylpropylènediamine pour obtenir le composé de formule :

**CH₂=CH-CH₂-O-CH₂-CH(OH)CH₂NHCH₂CONH(CH₂)₃N(Me)₂** (***X***)

ou

**R_{F}-A-NHCH₂CONH(CH₂)₃N(Me)₂** **(*XI*)**

avec **A = -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-**

Les composés (***X***) et (***XI***) sont les précurseurs des tensioactifs perfluoroalkylés cationiques et amphotères selon l'invention. Ces derniers sont obtenus , dans le cas des cationiques de type oxyde d'amine, au moyen de la perfluoroalkylation du groupement allylique des composés (***X***) à l'aide des iodures de perfluoroalcane, suivi de l'action du peroxyde d'hydrogène dans le 2-propanol, ou directement par action du peroxyde d'hydrogène sur les composés (***XI***) dans le 2-propanol ; dans le cas des amphotères de type carboxybétaïnique et sulfobétaïnique, par l'action du monochloroacétate de sodium, de l'acide acrylique ou du 3-chloro 2-hydroxypropanesulfonate de sodium sur les composés (***X***) et (***XI***) dans le 2-propanol, puis par perfluoroalkylation de la double liaison allylique à l'aide des iodures de perfluoroalcane lorsque cela est nécessaire.

Les mélanges obtenus sont ensuite dilués avec une petite quantité de monobutyle éther du diéthylène glycol et complétés avec de l'eau de telle sorte que la matière active des tensioactifs perfluoroalkylés de type aminoxyde soit d'environ 39% avec une teneur en fluor d'environ 11,5%, et celle des tensioactifs perfluoroalkylés de type carboxybétaïnique ou sulfobétaïnique soit d'environ 30% avec une teneur en fluor d'environ 9%.

Le pH des mélanges est réglé vers 7 ± 0,5.

Les tensioactifs perfluoroalkylés de type cationique et amphotère correspondant à
**A = CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-**
**B = S-(CH₂)_{q}-**
**D = -CO-NH-(CH₂)ᵣ-⁺N(R₁)(R₂)-O⁻, -(CH₂)_{q}-⁺N(R₁)(R₂)-(CH₂)ₛCOO⁻, -(CH₂)_{q}-⁺N(R₁)(R₂)-CH₂CH(OH)CH₂SO₃⁻,**
sont obtenus en trois ou quatre étapes : On fait réagir le 1-allyloxy-2,3 époxypropane ou les époxydes perfluoroalkylés de formules suivantes : 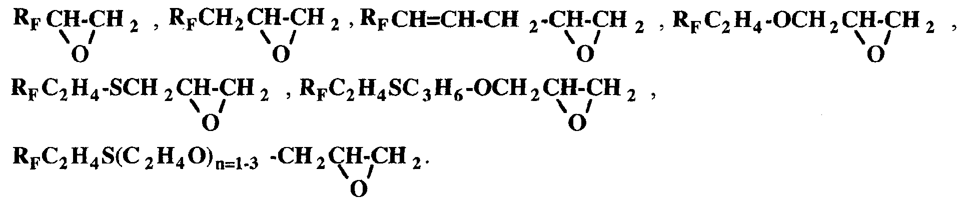 avec le thioglycolate de méthyle ou d'éthyle ou le 3-mercaptopropionate de méthyle ou d'éthyle. Le composé obtenu a pour formule :

**CH₂=CH-CH₂O-CH₂CH(OH)CH₂S(CH₂)_{q}COOR₅** **(*V*)**

ou **R_{F}-A-S(CH₂)_{q}COOR₅** (***VII***)

avec **A = - CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-**
avec q = 1 ou 2 et R₅ = Me ou Et

La fonction ester des composés (***V***) et **(*VII*)** est soumise à une réaction de transamidation que l'on effectue avec une diamine comme **H₂N(CH₂)ₚN(alkyle mineur)₂;** à titre d'exemple on peut utiliser la N,N-diméthylpropylènediamine pour obtenir le composé de formule :

**CH₂ = CH-CH₂OCH₂CH(OH)CH₂.S(CH₂)ᵣCONH(CH₂)₃N(Me)₂** **(*XII*)**

ou **R_{F}-A-S(CH₂)ᵣCONH(CH₂)₃N(Me)₂** (***XIII***)

avec **A = - CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-,** de l'acide acrylique ou du 3-chloro2-hydroxypropane sulfonate de sodium dans le 2-propanol suivie de la perfluoroalkylation de la double liaison allylique à l'aide des iodures de perfluoroalcane lorsque cela est nécessaire. Les mélanges obtenus sont ensuite dilués avec une petite quantité de monobutyléther du diéthylèneglycol et complétés avec de l'eau de sorte que la matière active des tensioactifs perfluoroalkylés de type carboxybétaïnique ou sulfobétaïnique soit d'environ 27% avec une teneur en fluor d'environ 9%. Le pH des mélanges est réglé vers 7 ± 0,5.

On remarquera que les enchaînements **-CH₂CH(I)CH₂-** et **-CH=CH-CH₂-** éventuellement contenus dans **A** sont une description idéale, car selon le caractère acide ou basique du milieu réactionnel un pourcentage variable d'iode contenu dans la molécule pourra être éliminé pour conduire à un mélange de ces deux enchaînements.

Tous les tensioactifs perfluoroalkylés de l'invention sont utilisables dans la technique comme agents hydrophobes et oléophobes ou comme additifs dans les mousses extinctrices pour améliorer leur résistance anti-incendie.

Les exemples suivants illustrent les procédés de préparation des nouveaux tensioactifs perfluoroalkylés de la présente invention sans toutefois en limiter la portée. Les parties et les pourcentages mentionnés sont en poids sauf indication contraire.

### Exemple 1

Préparation d'un tensioactif perfluoroalkylé anionique de type carboxylate correspondant à :

**R_{F} = C₆F₁₃**

**A = - CH₂CH(I)CH₂OCH₂CH(OH)CH₂ et/ou -CH=CH-CH₂OCH₂-CH(OH)CH₂-**
**B = -S-CH₂-**
**D = - CONH-CH₂COO⁻K⁺**

Dans un réacteur muni d'un réfrigérant et d'un agitateur, on introduit 6,84 pp (60 mmole) de 1-allyloxy-2,3-époxypropane et quelques gouttes d'une solution d'hydroxyde de benzyltriméthylammonium à 40% dans le méthanol. On ajoute ensuite lentement 6,36 pp (60 mmole) de thioglycolate de méthyle à 40°C , puis le mélange est chauffé à 60°C pendant 6 heures. Lorsque le mélange est revenu à température ambiante on ajoute 10 pp de 2-propanol, 7,53 pp (60 mmole) de chlorhydrate de glycinate de méthyle et 3,74 pp d'hydroxyde de potassium à 90% pour libérer le glycinate de méthyle de sa forme chlorhydrate puis on chauffe le mélange entre 85-90°C pendant 4 heures. Le mélange est ensuite refroidi vers 60°C. On ajoute alors 10 pp d'eau, 24,4 pp de iodure de perfluorohexane (54,7 mmole), 0,5 pp de 2,2'-azobis(2-isobutyronitrile) et 6 pp d'hydrosulfite de sodium dissous dans 19 pp d'eau. La réaction étant exothermique, la température s'élève vers 70-75°C ; on maintient la température du mélange entre 75 et 80°C pendant 3 heures. Lorsque le mélange a refroidi vers 50°C, on ajoute 20 pp d'eau et 7,7 pp d'hydroxyde de potassium à 90% dissout dans 13,4 pp d'eau. Le mélange est chauffé à 75°C pendant 4 heures puis refroidi entre 25 et 30°C. On ajoute alors 12 pp de monobutyléther du diéthylèneglycol . La matière active en composé fluoré représente environ 30% avec une teneur en fluor d'environ 9%.

### Exemple 2

Préparation d'un tensioactif perfluoroalkylé anionique de type carboxylate correspondant à :

**R_{F} = C₆F₁₃**

**A = - CH₂CH(I)CH₂OCH₂CH(OH)CH₂ et/ou -CH=CH-CH₂OCH₂-CH(OH)CH₂-**
**B = S-CH₂**
**D = - CON(Me)-CH₂COO⁻K⁺**

Dans un réacteur muni d'un réfrigérant et d'un agitateur, on introduit 6,84 pp (60 mmole) de 1-allyloxy-2,3-époxypropane et quelques gouttes d'une solution d'hydroxyde de benzyltriméthylammonium à 40% dans le méthanol. On ajoute ensuite lentement 6,36 pp (60 mmole) de thioglycolate de méthyle à 40°C puis le mélange est chauffé à 60°C pendant 6 heures. On ajoute ensuite 5,34 pp (60 mmole) de sarcosine et le mélange est chauffé entre 85 et 90°C pendant 4 heures. Le mélange est refroidi. Lorsque la température du mélange atteint 60°C , on ajoute 10 pp d'eau, 24,4 pp d'iodure de perfluorohexane (54,7 mmole), 0,5 pp de 2,2'-azobis(isobutyronitrile) et 6 pp d'hydrosulfite de sodium dissous dans 19 pp d'eau. La réaction est exothermique, la température s'élève vers 65 - 70°C ; on maintient la température du mélange entre 75 et 80°C pendant 3 heures. Lorsque le mélange a refroidi vers 50°C on ajoute 20 pp d'eau et 7,7 pp d'hydroxyde de potassium à 90% dissous dans 13,4 g d'eau. Le mélange est chauffé à 75°C pendant 4 heures puis refroidi entre 25 et 30°C. On ajoute alors 12 pp de monobutyléther du diéthylène glycol et on complète le mélange avec 16,46 pp d'eau de telle sorte que la matière active en composé fluoré représente environ 30% avec une teneur en fluor d'environ 9%.

### Exemple 3

Préparation d'un tensioactif perfluoroalkylé anionique de type carboxylate correspondant à :

**R_{F}** = **C₆F₁₃**

**A = -CH₂-CH(I) CH₂- et/ou CH=CH-CH₂-**
**B = néant**
**D = -NHCO-(CH₂)₂-COO⁻K⁺**

Dans un réacteur muni d'un réfrigérant et d'un agitateur, on introduit 6 pp d'anhydride succinique et 10 pp de diméthylformamide. On ajoute ensuite lentement 3,42 pp d'allylamine. La réaction est exothermique. La température s'élève vers 45°C. On maintient le mélange à cette température pendant une demi-heure puis on chauffe entre 40 et 50°C pendant 1 heure. Le mélange est ensuite chauffé à 60°C puis on ajoute 14,4 pp d'iodure de perfluorohexane (54,7 mmole), 0,5 pp de 2,2'-azobis (isobutyronitrile) et 6 pp d'hydrosulfite de sodium dissous dans 19 pp d'eau et on chauffe ensuite entre 75 et 80°C pendant 3 heures. Lorsque le mélange est revenu à température ambiante, on ajoute 3,74 g d'hydroxyde de potassium à 90% dissous dans 6,68 g d'eau. On ajoute alors 10,4 pp de monobutyléther du diéthylène glycol et on complète le mélange avec 63,86 pp d'eau de telle sorte que la matière première en composé fluoré représente environ 25% avec une teneur en fluor d'environ 9%.

### Exemple 4

Préparation d'un tensioactif perfluoroalkylé cationique de type oxyde d'amine correspondant à:

**R_{F}** = **C₆F₁₃**

**A = -CH₂CH(I)CH₂OCH₂CH(OH)CH₂ et/ou -CH=CH-CH₂OCH₂-CH(OH)CH₂-**
**B = -S-CH₂-**
**D = CONH- (CH₂)₃-⁺N(Me)₂-O⁻**

Dans un réacteur muni d'un réfrigérant et d'un agitateur, on introduit 6,84 pp (60 mmole) de 1-allyloxy -2,3-époxypropane et quelques gouttes d'une solution d'hydroxyde de benzyltriméthylammonium à 40% dans le méthanol. On ajoute ensuite lentement 6,36 pp (60 mmole) de thioglycolate de méthyle à 40°C puis le mélange est chauffé à 60°C pendant 6 heures. On maintient la température du mélange à 60°C et on ajoute 6,12 pp (60 mmole) de N,N-diméthylpropylène diamine puis on chauffe à 90°C pendant 4 heures. Le mélange est ensuite refroidi vers 60°C. On ajoute alors 10 pp d'eau, 24,4 pp d'iodure de perfluorohexane (54,7 mmole), 0,5 pp de 2,2'-azobis (isobutyronitrile) et 6 pp d'hydrosulfite de sodium dissous dans 19 pp d'eau. Le mélange est amené à température ambiante ; on introduit alors 10 pp de 2-propanol et on ajoute 9,1 pp de peroxyde d'hydrogène suffisamment lentement pour que la température du mélange ne dépasse pas 60°C. Le mélange est chauffé à 75°C pendant 4 heures puis refroidi entre 25 et 30°C. On ajoute alors 12 pp de monobutyléther du diéthylène glycol et on complète le mélange avec 8,68 pp d'eau de telle sorte que la matière active en composé fluoré représente environ 39% avec une teneur en fluor d'environ 11,6%.

### Exemple 5

Préparation d'un tensioactif perfluoroalkylé amphotère de type carboxybétaïne correspondant à :

**R_{F} - C₆F₁₃**

**A = -CH₂CH(I)CH₂OCH₂CH(OH)CH₂ et/ou -CH=CH-CH₂OCH₂-CH(OH)CH₂-**
**B = -⁺N(Me)₂--(CH₂)₃-**
**D = NHCO-(CH₂)₂COO⁻**

Dans un réacteur muni d'un mélangeur et réfrigérant, on introduit 6 pp (60 mmole) d'anhydride succinique et 10 pp de diméthylformamide. Puis 6,12 pp (60 mmole) de N,N-diméthylpropylènediamine sont ajoutées lentement au mélange à 30°C. Lorsque l'addition est terminée, la température du mélange s'élève jusqu'à 80°C. Le mélange est ensuite chauffé à 80°C pendant 2 heures. Le mélange est refroidi vers 60°C. On ajoute alors 10 pp de 2-propanol et 6,84 pp (60 mmole) de 1-allyloxy-2,3-époxypropane. La réaction est légèrement exothermique. Le mélange est chauffé à 85°C pendant 3 heures. On laisse revenir la température à 60°C puis on ajoute successivement 24,4 pp d'iodure de perfluorohexane (54,7 mmole), 0,5 pp de 2,2'-azobis(isobutyronitrile) et 6 pp d'hydrosulfite de sodium dissous dans 19 pp d'eau. La réaction est exothermique. La température s'élève vers 70-75°C. On maintient la température entre 70 et 80°C pendant 3 heures. Le mélange est ensuite refroidi vers 25-30°C et on ajoute 11 pp de monobutyléther du diéthylène glycol puis on complète le mélange avec 52,14 pp d'eau de telle sorte que la matière active en composé fluoré représente environ 30% avec une teneur en fluor d'environ 9%.

### Exemple 6

Préparation d'un tensioactif perfluoroalkylé amphotère de type carboxybétaïne correspondant à:

**R_{F}** = **C₆F₁₃ (43%), C₈F₁₇ (35%), C₁₀F₂₁ (15%), C₁₂F₂₅ (7%).**

**A = - CH₂CH(I)CH₂OCH₂CH(OH)CH₂ et/ou -CH=CH-CH₂OCH₂-CH(OH)CH₂-**
**B = -⁺N(Me)₂-(CH₂)₃-**
**D = -NHCO-(CH₂)₂COO⁻**

Le procédé de préparation est identique à celui décrit dans l'exemple 5 , mais au lieu d'utiliser l'iodure de perfluorohexane comme agent perfluoroalkylant, on utilise un iodure de perfluoroalcane constitué d'un mélange de chaînes perfluorées. Le mélange obtenu a une matière active en composé fluoré d'environ 27% avec une teneur en fluor d'environ 9,5 %.

### Exemple 7

Préparation d'un tensioactif perfluoroalkylé amphotère de type carboxybétaïne correspondant à:

**R_{F} = C₆F₁₃**

**A = - CH₂CH(I)CH₂OCH₂CH(OH)CH₂ et/ou -CH=CH-CH₂OCH₂-CH(OH)CH₂-**
**B = -S -CH₂-**
**D = -CONH -(CH₂)₃-⁺N(Me)2-CH₂COO⁻**

Dans un réacteur muni d'un réfrigérant et d'un agitateur, on introduit 6,84 pp (60 mmole) de 1-allyloxy-2,3-époxypropane et quelques gouttes d'une solution d'hydroxyde de benzyltriméthylammonium à 40% dans le méthanol. On ajoute ensuite lentement 6,36 pp (60 mmole) de thioglycolate de méthyle à 40°C puis le mélange est chauffé à 60°C pendant 6 heures. On maintient la température du mélange à 60°C et on ajoute 6,12 pp (60 mmole) de N,N-diméthylpropylène diamine puis on chauffe à 90°C pendant 4 heures. Le mélange est ensuite refroidi vers 50°C et on ajoute 10 pp de 2-propanol puis 7,7 pp de monochloroacétate de sodium. Le mélange est chauffé à 80°C pendant 7 heures puis refroidi vers 60°C. On ajoute alors successivement 4,14 pp de carbonate de potassium dissous dans 5 pp d'eau, 24,4 pp d'iodure de perfluorohexane (54,7 mmole), 0,5 pp de 2,2'-azobis (isobutyronitrile) et 6 pp d'hydrosulfite de sodium dissous dans 19 pp d'eau. La réaction est exothermique. La température s'élève vers 70-75°C. On maintient la température entre 75 et 80°C pendant 3 heures. Le mélange est ensuite refroidi vers 25-30°C et on ajoute 12 pp de monobutyl éther du diéthylène glycol puis on complète le mélange avec 43,94 pp d'eau de telle sorte que la matière active en composé fluoré représente environ 30% avec une teneur en fluor d'environ 9%.

### Exemple 8

Préparation d'un tensioactif perfluoroalkylé amphotère de type carboxybétaïne correspondant à:

**R_{F} = C₆F₁₃**

**A = - CH₂CH(I)CH₂OCH₂CH(OH)CH₂ et/ou -CH=CH-CH₂OCH₂-CH(OH)CH₂-**
**B = -S-CH₂-**
**D = -CONH -(CH₂)₃-⁺N(Me)₂-(CH₂)₂COO⁻**

Le procédé de préparation de ce composé est identique à celui de l'exemple 7. Au lieu d'utiliser le monochloracétate de soude comme agent de quaternisation, on utilise l'acide acrylique (4,4 pp/61 mmole). La matière active en composé fluoré du mélange obtenu représente environ 31 % avec une teneur en fluor d'environ 9%.

### Exemple 9

Préparation d'un tensioactif perfluoroalkyle amphotère de type sulfobétaïne correspondant à :

**R_{F} = C₆F₁₃**

**A = -CH₂CH(I)CH₂OCH₂CH(OH)CH₂ et/ou -CH=CH-CH₂OCH₂-CH(OH)CH₂-**
**B = -S-CH₂-**
**D = -CONH-(CH₂)₃-⁺N(Me)₂-CH₂CH(OH)CH₂SO₃⁻**

Le procédé de préparation de ce composé est identique à celui décrit dans l'exemple 8. Au lieu d'utiliser l'acide acrylique comme agent de quaternisation, on utilise le 3-chloro-2-hydroxy-1-propane sulfonate de sodium (13pp/66 mmole). La matière active en composé fluoré du mélange obtenu représente environ 33% avec une teneur en fluor d'environ 9%.

### Exemple 10

Préparation d'un tensioactif perfluoroalkylé amphotère de type carboxybétaïne correspondant à:

**R_{F} = C₆F₁₃**

**A = - CH₂CH(I)CH₂OCH₂CH(OH)CH₂ et/ou -CH=CH-CH₂OCH₂-CH(OH)CH₂-**
**B = -NH-CH₂-**
**D = -CONH - (CH₂)₃-⁺N(Me)₂-CH₂COO⁻**

Dans un réacteur muni d'un mélangeur et d'un réfrigérant, on introduit 7,53 pp (60 mmole) de chlorhydrate de glycinate de méthyle, 10 pp de 2-propanol et 3,74 pp d'hydroxyde de potassium à 90%. On ajoute alors à 50°C, 6,84 pp (60 mmole) de 1-allyloxy-2,3-époxypropane. Le mélange est chauffé à 70°C pendant 6 heures. Le mélange est refroidi vers 60°C puis on ajoute 6,12 pp (60 mmole) de N,N-diméthylpropylène diamine et on chauffe entre 80 et 85°C pendant 4 heures. On laisse revenir la température à 60°C puis on ajoute successivement 10 pp d'eau, 24,4 pp d'iodure de perfluorohexane , 0,5 pp de 2,2'-azobis(isobutyronitrile) et 6 pp d'hydrosulfite de sodium dissous dans 19 pp d'eau. La réaction est exothermique. La température s'élève vers 75-80°C. On maintient la température entre 75-80°C pendant 3 heures. Le mélange est ensuite refroidi vers 50-60°C et on ajoute 7,7 pp (66 mmoles) de monochloracétate de sodium dissous dans 14 pp d'eau. Le mélange est chauffé à 80°C pendant 7 heures.

On laisse revenir la température du mélange vers 25-30°C et on ajoute 12 pp de monobutyléther du diéthylène glycol puis on complète le mélange avec 24,17 pp d'eau de telle sorte que la matière active en composé fluoré représente environ 30% avec une teneur en fluor d'environ 9%.

### Exemple 11

Tableau rassemblant les mesures de la tension superficielle et du pouvoir moussant des composés des exemples 1 à 10.

| Exemples | Tension superficielle* 0,1% (20°C) [mN/m] | Pouvoir moussant ** [mm] | |
|---|---|---|---|
| | | Immédiatement | après 5 minutes |
| 1 | 17,3 | 81 | 70 |
| 2 | 18,3 | 82 | 73 |
| 3 | 23,6 | 11 | 0 |
| 4 | 18,1 | 120 | 98 |
| 5 | 16,4 | 78 | 61 |
| 6 | 19,1 | 55 | 41 |
| 7 | 18,4 | 181 | 129 |
| 8 | 18,5 | 88 | 68 |
| 9 | 19,9 | 96 | 78 |
| 10 | 21,8 | 8 | 0 |

| | | | |
|---|---|---|---|
| * Mesurée selon la méthode de l'anneau ** Déterminée selon le procédé de Ross-Miles avec des solutions aqueuses à 0,1% à 25°C. | | | |

## Revendications

1. **R_{F}A-B-D, caractérisés par le fait qu'**ils sont hydrophobes et qu'ils contiennent une fonction amide substituée directement liée à ou séparée de la chaîne polyfluoroalkylée par un groupement **-N⁺R₁R₂-(CH₂)ₚ-, -S-(CH₂)_{q}-** ou **-NH -(CH₂)_{q}-** où
**R_{F}** = chaîne perfluoroalkyle linéaire ou ramifiée **CₙF₂ₙ₊₁** où **n = 4 à 20,** ou un mélange de groupements perfluoroalkyle linéaires ou ramifiés ;
**A = -CH₂CH(I)CH₂-, -CH=CHCH₂-, -CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂- ;**
**B = -S-(CH₂)q-, -NH(CH₂)_{q}-** ou **-N⁺R₁R₂-(CH₂)p-** lorsque **A = -CH₂CH(I)CH₂OCH₂CH (OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃ CH₂CH(OH)CH₂-, -C₂H₄S-C₃H₆OCH₂CH(OH)CH₂-, Néant** lorsque **A = -CH₂CHICH₂-, -CH=CHCH₂- ;**
**D = -CONH-(CH₂)ᵣN⁺R₁R₂-E, -CONR₃(CH₂)ₛCOOM** lorsque B **= -S-(CH₂)_{q}-, -NH(CH₂)_{q}- ; -NHCO-(CH₂)ᵣCOO⁻, -NHCO-CH(SO₃M)CH₂COO⁻,** +
lorsque **B = -N⁺R₁R₂-(CH₂)ₚ;**
**-NHCO-(CH₂)ᵣCOOM, -NHCO-CH(SO₃M)CH₂COOM,**
lorsque **B = Néant ;**
**E = -(CH₂)ₛCOO⁻, -O⁻, -CH₂CHOHCH₂SO₃⁻;**
**M = un ion alcalin;**
**p, r = 2 ou 3;**
**q, s = 1 ou 2 ;**
**R₁, R₂ = groupements alkyle mineur identiques;**
**R₃ = H, CH₃.**

2. Procédé d'obtention des composés tensioactifs perfluoroalkylés selon la revendication (1) **caractérisé par le fait que** l'amide substituée substitué correspondant à **D** contenant la séquence **-CONH-,** est obtenue soit par ouverture du cycle époxydique du 1-allyloxy-2,3-époxypropane ou des époxydes perfluoroalkylés de formules suivantes 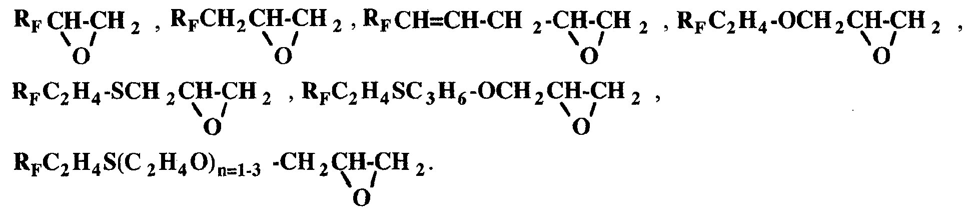 par des α et β thio- et amino-esters qui sont soumis à une réaction de transamidation :
- soit par des N,N-dialkylalkylènediamines suivie de la transformation du groupement amine tertiaire des intermédiaires obtenus en ammonium quaternaire par des agents de quaternisation,
- soit par des α et β thioesters, α et β amino-esters ou α et β amino-acides.

3. Procédé d'obtention des composés tensioactifs perfluoroalkylés, la revendication (2) **caractérisé par le fait que** les α et P thio-esters sont le glycolate de méthyle ou d'éthyle et le 3-mercaptopropionate de méthyle ou d'éthyle, que les α et β amino-esters sont le glycinate de méthyle ou d'éthyle et le 3-aminopropionate de méthyle ou d'éthyle, et que les α et β amino-acides sont la glycine ou la sarcosine.

4. Procédé d'obtention des composés tensioactifs perfluoroalkylés selon les revendications (2) et (3) **caractérisé par le fait que** les agents quaternisants sont le monochloracétate de sodium, l'acide acrylique, le 3-chloro 2-hydroxy propane sulfonate de sodium, le peroxyde d'hydrogène.

5. Procédé d'obtention des composés tensioactifs perfluoroalkylés selon la revendication (1) **caractérisé par le fait que** l'amide substituée correspondant à **D** contenant la séquence - **NHCO-** est obtenue soit par réaction d'une N,N-dialkylalkylènediamine avec un anhydride d'acide (si l'anhydride d'acide est l'anhydride maléique, sa double liaison peut être préalablement soumise à une réaction de sulfonation) suivie de la quaternisation du groupement amine tertiaire par un époxyde comportant une double liaison terminale capable de réagir avec un iodure de perfluoroalcane ou un mélange de iodures de perfluoroalcanes ou par un époxyde perfluoroalkylé , soit par réaction de l'allylamine avec un anhydride d'acide (si l'anhydride d'acide est l'anhydride maléique, sa double liaison peut être préalablement soumise à une réaction de sulfonation).

6. Procédé d'obtention des composés tensioactifs perfluoroalkylés selon la revendication (5) **caractérisé par le fait que** les anhydrides d'acide sont ceux des acides succinique, glutarique, maléique.

7. Procédé d'obtention des composés tensioactifs perfluoroalkylés selon les revendications (5) et (6) **caractérisé par le fait que** la N,N-dialkylalkylènediamine est la N,N-dialkyl éthylènediamine, la N,N éthyléthylènediamine et de préférence la N,N-diméthylpropylène diamine ou la N,N-diéthylpropylènediamine.

8. Procédé d'obtention des composés tensioactifs perfluoroalkylés selon les revendications (5), (6) et (7) **caractérisé par le fait que** l'époxyde utilisé pour la réaction de quaternisation est le 1-allyloxy-2,3 époxypropane ou les époxydes de formules suivantes :

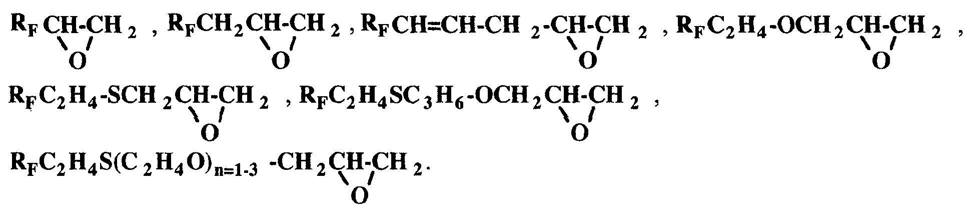

9. Composés tensioactifs perfluoroalkylés selon la revendication (1) **caractérisés par le fait que** la double liaison allylique terminale de tous les composés de l'invention est soumise à une réaction d'addition avec un iodure de perfluoroalcane ayant 4 à 20 atomes de carbone ou un mélange d'iodures et de perfluoroalcanes.

10. Utilisation de composés tensioactifs perfluoroalkylés selon la revendication (1) comme agents hydrophobes et oléophobes ou comme additifs dans les mousses extinctrices pour améliorer leur résistance anti-incendie.

## Patentansprüche

1. Perfluoralkylisierte Tenside der folgenden allgemeinen Formel:
**R_{F} A - B - D,** diese sind **dadurch gekennzeichnet, dass** sie hydrophobe und oleophobe sind, dass sie eine substituierte Amidgruppe enthalten, die direkt an die perfluoralkylisierten Gruppen gebunden ist, oder die von der perfluoroalkylisierten Guppe durch eine Gruppe - **N⁺R₁R₂-(CH₂)ₚ-, -S-(CH₂)_{q}-** ou **-NH-(CH₂)_{q}-** abgetrennt ist,
wo
**R_{F}** = perfluoralkylisierte gerade oder verzweigte Kette wo n= 4 bis 20 , oder ein Gemisch aus den geraden oder verzweigten perfluoralkylisierten Gruppen ;
**A** = **-CH₂CH(I)CH₂-, -CH=CHCH₂-, -CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₃CH(OH)CH₂-, -C₂H₄S(C₂H₄O)ₙ₌₁₋₃CH₂CH(OH)CH₂-, -C₂H₄S-C₃H₆OCH₂CH(OH)CH₂- ;**
**B = -S-(CH₂)_{q}-, -NH(CH₂)_{q}** oder **-N⁺R₁R₂-(CH₂)ₚ** wenn
**A = -CH₂CH(I)CH₂OCH₂CH(OH)CH₂-, -CH=CH-CH₂OCH₂CH(OH)CH₂-, - CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃ CH₂CH(OH)CH₂-, -C₂H₄S-C₃H₆OCH₂CH(OH)CH₂-,** Nichts
wenn **A = -CH₂CHICH₂-, -CH=CHCH₂-;**
**D** = **-CONH-(CH₂)ᵣN⁺R₁R₂-E, -CONR₃(CH₂)₆COOM**
wenn **B = -S-(CH₂)_{q}-, -NH(CH₂)_{q}- ;**
**-NHCO-(CH₂)ᵣCOO⁻, -NHCO-CH(SO₃M)CH₂COO⁻,**
wenn **B = Nichts ;**
**E = -(CH₂)ₛCOO⁻, -O⁻, -CH₂CHOHCH₂SO₃⁻;**
**M = ein alkalisches Metallion;**
**p, r = 2 ou 3;**
**q, s = 1 ou 2 ;**
**R₁, R₂ =** kleinere gleiche **Alkyl-Gruppierungen;**
**R₃ = H, CH₃ ;**

2. Verfahren zur Herstellung von perfluoroalkylisierten Tensiden nach Anspruch (1), **dadurch gekennzeichnet, dass** die substituierte Amidgruppe korrespondierend zu **D**, die die Sequenz **-CONH-** enthält, erfolgt entweder durch eine Ringöffsnungreaktion des 1-Allyloxy-2,3-Epoxypropans oder perfluoroalkylisierten Epoxiden der folgenden Formeln: 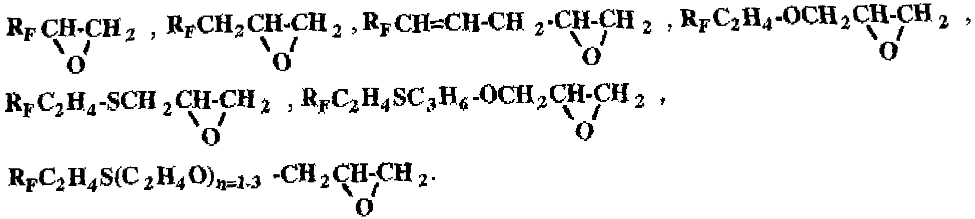 mit α und β Thio- und Amino-ester, die einer Transamidierung-reaktion unterworfen werden:
- oder durch N,N-Dialkylalkylenediaminen, gefolgt von der Umwandlung der tertiären Aminogruppe, der in quaternären Ammonium durch Quaternisierungsmittel gebildeten Zwischenprodukten,
- oder durch α und β Thioestern, α und β Amino-estern oder α und β Aminosäuren.

3. Verfahren zur Herstellung von perfluoroalkylisierten Tensiden nach Anspruch (1) und (2), **dadurch gekennzeichnet, dass** die α und β Thioestern sind Methyl oder Ethyl Glykolat und Methyl oder Ethyl 3-Mercaptopropionat, dass die α und β Amino-estern sind Methyl oder Ethyl Glycinat und Methyl oder Ethyl 3-Aminopropionat, und dass die α und β Aminosäuren Glycin und Sarcosin sind.

4. Verfahren zur Herstellung von perfluoroalkylisierten Tensiden nach Anspruch (2) und (3), **dadurch gekennzeichnet, dass** die Quaternisierungsmittel Natrium Chloroacetat, Acrylsäure, Natrium 3-Chloro 2-Hydroxy Propan Sulfonat, Wassesoffperoxid sind.

5. Verfahren zur Herstellung von perfluoroalkylisierten Tensiden nach Anspruch (1), ***dadurch* gekennzeichnet dass** die substituierte Amidgruppe korrespondierend zu **D,** die die Sequenz **-CONH-** enthält, entweder durch Umsetzung einer N,N-dialkylalkylenediamin mit einem Säureanhydrid (wenn Säureanhydrid Maleinsäureanhydrid ist, wird seine *Doppelbindung zuvor sulfoniert) gefolgt von der Quaternisierung* der tertiären Aminogruppe mit einem Epoxid, die eine endständige Doppelbindung enthält, die mit einem 1-Iodperfluoroalkan oder mit einem Gemisch von 1-Iodoperfluoroalkanen reagieren kann oder mit einem perfluoroalkylisierten Epoxid, oder durch Umsetzung der Allylamin mit einem Saüreanhydrid (wenn Säureanhydrid Maleinsäureanhydrid ist, wird seine Doppelbindung zuvor sulfoniert).

6. Verfahren zur Herstellung von perfluoroalkylisierten Tensiden nach Anspruch (1) und (5), **dadurch gekennzeichnet, dass** die Säureanhydriden, Glutarssäureanhydrid, Maleinsäureanhydrid sind.

7. Verfahren zur Herstellung von perfluoroalkylisierten Tensiden nach Anspruch (5) und (6), **dadurch gekennzeichnet, dass** die N,N-Dialkylalkylendiamin N,N-Dialkylalkylendiamin, N,N Ethylethylendiamine und vorzugsweise N,N-Dimethylpropylendiamin oder N,N-Diethylpropylendiamin ist.

8. Verfahren zur Herstellung von perfluoroalkylisierten Tensiden nach Anspruch (5), (6) und (7) **dadurch gekennzeichnet, dass** der Epoxid, die für den Quaternierungreaktion verwendet wird, 1-Allyloxy-2,3 Epoxypropan oder perfluoroalkylisierten Epoxiden der folgenden Formeln ist:

9. Verfahren zur Herstellung von pert'luoroallCylisiexten Tensiden nach Anspruch (1), **dadurch gekennzeichnet, dass** die endständige allylische Doppelbindung, der aller Verbindungen der Erfindung einer Additionsreaktion mit einem 1-Iodperfluoroalkan oder einem Gemisch von 1-iodoperfluoroalkanen unterworfen wird.

10. Verwendung von perfluoroalkylisierten Tensiden nach Anspruch (1) als Hydrophobierung- und Oleophobierungsmittel oder als Zusatzmittel für die Feuerlöschschäumen, um deren Widerstandsfähigkeit zu verbessern.

## Claims

1. Perfluoroalkyl surfactants corresponding to the following general formula:
**R_{F} A - B** - **D**, **characterised by** the fact they are hydrophobic and they contain a substituted amide function rirectly linked to or separated from the polyfluoroalkylated chain by a group **-N⁺R₁R₂-(CH₂)ₚ-, -S-(CH₂)_{q}-** ou **-NH-(CH₂)_{q}-** where
**R_{F} =** linear or branched perfluoroalkyl chain **CₙF₂ₙ₊₁** where **n = 4 à 20,** or a mixture of linear or branched perfluoroalkyl groups ;
**A = -CH₂CH(I)CH₂-, -CH=CHCH₂-, -CH₂CH(I)CH₂OCH₂CH(OH)CH₂-, -CH=CH-CH₂OCH₂CH(OH)CH₂-, -CH(OH)CH₂-, -CH₂CH(OH)CH₂-, -CH=CHCH₂CH(OH)CH₂-, -C₂H₄OCH₂CH(OH)CH₂-, -C₂H₄SCH₂CH(OH)CH₂-, -C₂H₄S(C₂H₄O)ₙ₌₁₋₃CH₂CH(OH)CH₂-, -C₂H₄S-C₃H₆OCH₂CH(OH)CH₂-;**
**B = -S-(CH₂)_{q}-, -NH(CH₂)_{q}- or -N⁺R₁R₂- (CH₂)ₚ-** when
**A = -CH₂CH(I)CH₂OCH₂CH(OH)CH₂-, -CH=CH-CH₂OCH₂CH(OH)CH₂-, - CH(OH)CH₂-, -CH₂CH(OH)CH₂-, -CH=CHCH₂CH(OH)CH₂-, -C₂H₄OCH₂CH(OH)CH₂-,-C₂H₄SCH₂CH(OH)CH₂-, -C₂H₄S(C₂H₄O)ₙ₌₁₋₃ CH₂CH(OH)CH₂-, -C₃H₄S-C₃H₆OCH₂CH(OH)CH₂-, None**
when **A = -CH₂CHICH₂-, -CH=CHCH₂-;**
**D = -CONH-(CH₂)ᵣN⁺R₁R₂-E, -CONR₃(CH₂)₃COOM**
when **B = -S-(CH₂)_{q}-, -NH(CH₂)_{q}- ;**
- **NHCO-(CH₂)ᵣCOO⁻, -NHCO-CH(SO₃M)CH₂COO⁻,**
**when B** = **-N⁺R₁R₂-(CH₂)ₚ;**
- **NHCO-(CH₂)ᵣCOOM, -NHCO-CH(SO₃M)CH₂COOM,**
when **B = None ;**
**E = -(CH₂)₈COO⁻, -O⁻, -CH₂CHOHCH₂SO₃-;**
**M = alkali metal;**
**p, r = 2 or 3 ;**
**q, s = 1 or 2 ;**
**R₁, R₂ = identical minor alkyl groups;**
**R₃ = H, CH₃;**

2. Process for obtaining perfluoroalkylated surfactants according to the claim (1) **characterised by** the fact that the substituted amide corresponding to **D** containing the sequence **-CONH-,** is obtained either by opening the epoxy ring of 1-allyloxy-2,3-epoxypropane or perfluoroalkylated epoxydes with the following formulas : 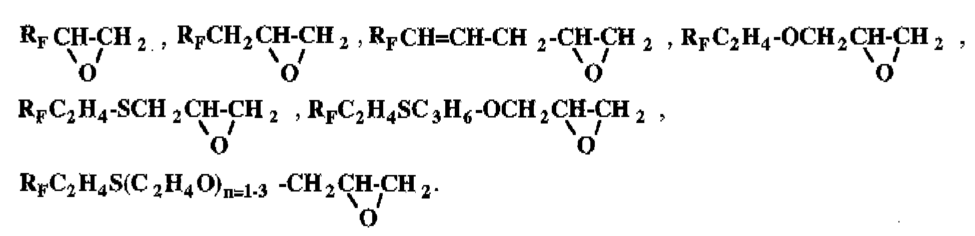 by α and β thio- et amino-esters which are subjected to a transamidation reaction :
- or by N,N-dialkylalkylenediamines followed by the conversion of tertiary amine group of intermediates obtained in quaternary ammonium by means of quaternizing agents,
- or by α and β thioesters, α and β amino-esters or α and β amino-acids.

3. Process for obtaining perfluoroalkylated surfactants according to the claim (2) **characterised by** the fact that the α and β thio-esters are the methyl or ethyl glycolate and the methyl or ethyl 3-mercaptopropionate, that the α and β amino-esters are the methyl or ethyl glycinate and the methyl or ethyl 3-aminopropionate, and that the α and β amino-acids are the glycine or sarcosine.

4. Process for obtaining perfluoroalkylated surfactants according to the claim (2) and (3) **characterised by** the fact that the quaternizing agents are the sodium monochloroacetate, the acrylic acid, the sodium 3-chloro 2-hydroxy propane sulfonate, the hydrogen peroxide.

5. Process for obtaining perfluoroalkylated surfactants according to the claim (1) **characterised by** the fact that the substituded amide corresponding to D containing the sequence **-CONH-** is obtained either by reaction of a N,N-dialkylalkylènediamine with an acid anhydride (if the acid anhydride is maleic anhydride, its double bond may be prealably subjected to a sulfonation reaction) followed by quaternizing of tertiary amine group with an epoxide comprising a terminal double bond cabable of reacting with a perfluoroalkane iodide or a mixture of perfluoralkane iodides or with a perfluoroalkylated epoxide, or by reaction of the allylamine with an acid anhydride (if the acid anhydride is maleic anhydride, its double bond may be prealably subjected to a sulfonation reaction).

6. Process for obtaining perfluoroalkylated surfactants according to the claim (5) **characterised by** the fact that the acid anhydrides are succinic, glutaric, maleic anhydrides.

7. Process for obtaining perfluoroalkylated surfactants according to the claims (5) and (6) **characterised by** the fact that the N,N-dialkylalkylenediamine is the N,N-dialkyl ethylenediamine, the N,N-ethylethylenediamine and preferably the N,N-dimethylpropylene diamine or the N,N-diethylpropylenediamine.

8. Process for obtaining perfluoroalkylated surfactants according to the claims (5), (6) and (7) **characterised by** the fact that the epoxide used for the quaternizing reaction is the 1-allyloxy-2,3 epoxypropane or the epoxydes with the following formulas :

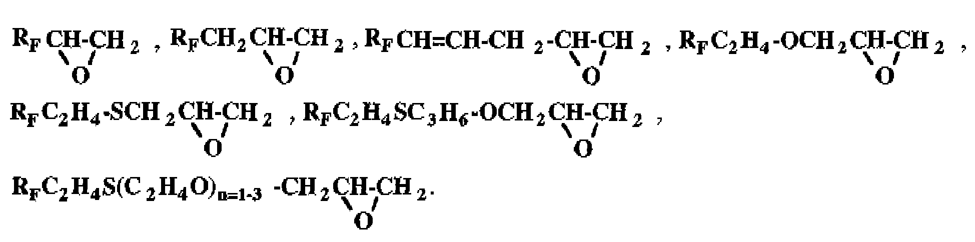

9. Process for obtaining perfluoroalkylated surfactants according to the claim (1), **characterised by** the fact that the terminal allylic double bond of all the compounds of the invention is subjected to an addition reaction with a perfluoroalkane iodide comprising 4 to 20 carbon atoms or a mixture of perfluoroalkane iodides.

10. Use of perfluoroalkylated surfactants according to the claim (1), as hydrophobic and oleophobic agents or as additives in the fire-fighting foams to improve their fire-fighting resistance.
